# EUROPÄISCHE PATENTSCHRIFT

(11) **EP 1 721 577 B1**
(45) Veröffentlichungstag und Bekanntmachung des Hinweises auf die Patenterteilung: **02.04.2008**
(21) Anmeldenummer: 06008584.2
(22) Anmeldetag: 26.04.2006
(51) Int. Cl.: A61B 17/32, A61B 17/28

(54) **Endoskopisches Instrument**
Endoscopic instrument
Instrument endoscopique

(30) Priorität: 09.05.2005 DE 102005021234
(43) Veröffentlichungstag der Anmeldung: 15.11.2006
(73) Patentinhaber: Karl Storz GmbH & Co. KG, 78532 Tuttlingen (DE)
(72) Erfinder: Berberich, Sascha, 78532 Tuttlingen (DE); Deufel, Egon, 78567 Fridingen (DE)
(74) Vertreter: Hofmeister, Frank

(56) Entgegenhaltungen:
- EP-A- 0 677 275
- WO-A-91/02493
- DE-U1- 8 302 716
- US-A- 4 258 716

## Beschreibung

Die Erfindung betrifft ein medizinisches Instrument mit einem hohlen Schaft, an dessen proximalem Ende eine aus mindestens zwei Griffteilen bestehende Handhabe angeordnet ist und an dessen distalem Ende ein aus mindestens zwei Maulteilen bestehendes Werkzeug angeordnet ist, wobei mindestens ein Maulteil des Werkzeugs zum Öffnen und Schließen über ein verschwenkbar ausgebildetes Griffteil der Handhabe gegenüber dem mindestens einen anderen Maulteil des Werkzeugs verstellbar ist und das mindestens eine verstellbare Maulteil und das entsprechende, zum Verstellen des Maulteils dienende Griffteil der Handhabe über eine axial verschiebbare in dem hohlen Schaft gelagerte Zug-/Druckstange miteinander verbunden sind.

Gattungsgemäße medizinische Instrumente, bei denen das bewegliche Maulteil ausgehend von einem verschwenkbaren Griffteil der Handhabe über ein Zug/Druckelement betätigbar ist, finden insbesondere in der endoskopischen Chirurgie vielseitige Verwendung, beispielsweise als Stanzen, Scheren, Nadelhalter, Fassinstrumente und dergleichen. Bei diesen bekannten Instrumenten sind die das Werkzeug bildenden Maulteile in der Regel in Längsrichtung des Instrumentenschaftes angeordnet, so dass mit diesen Instrumenten nur Operationsgebiete erreichbar sind, die direkt zugänglich im wesentlichen vor der Instrumentenspitze angeordnet sind. Da aber manche Operationsgebiete nicht auf direktem geradlinigen Weg zugänglich sind, sind diese bekannten Instrumente für diese Zwecke gar nicht, oder nur bedingt geeignet.

Weiterhin sind gattungsgemäße medizinische Instrumente bekannt, bei denen die Maulteile des Werkzeugs seitlich abgewinkelt zur Längsachse des Schaftes ausgerichtet sind. Da bei diesen Instrumenten das verschwenkbare Maulteil des Werkzeugs beim Öffnen und Schließen, relativ zur Axialbewegung der Zug-/Druckstange betrachtet, eine Drehbewegung ausführt, ist es notwendig, die über die Bewegung der Handhabe initiierte Axialbewegung der Zug-/Druckstange in eine Drehbewegung umzusetzen. Hierzu ist es aus der Praxis bekannt, die Handhabe und die Zug-/Druckstange über ein Zahnradgetriebe miteinander zu koppeln. Diese Ausgestaltung ist zwar relativ einfach herzustellen, jedoch genügt die Kraftübertragung auf das Werkzeugs nicht allen operativen Anforderungen.

Aus der DE 103 27 655 A1 ist ein gattungsgemäßes medizinisches Instrument mit seitlich zur Längsachse des Schaftes abgewinkelten Maulteilen bekannt, bei dem die Bewegungsumsetzung über einen Übertragungshebel erfolgt, der einerseits an der Zug-/Druckstange gelagert ist und andererseits mit dem verstellbaren Maulteil des Werkzeugs verbunden ist. Diese konstruktive Umsetzung der Axialbewegung in eine Drehbewegung hat sich in der Praxis durchaus bewährt, jedoch ist diese Ausgestaltung konstruktiv sehr aufwendig.

WO 91/02493 A1, US 4,258,716 A und DE 8 302 716 U1 offenbaren medizinische Instrumente, die die Merkmale der Präambel des Anspruchs 1 besitzen.

Davon ausgehend liegt der Erfindung die **Aufgabe** zugrunde, ein medizinisches Instrument der eingangs genannten Art bereitzustellen, bei dem die Umsetzung der Axialbewegung der Zug-/Druckstange in die Drehbewegung des verstellbaren Maulteils einfach und zuverlässig erfolgt.

Die **Lösung** dieser Aufgabenstellung ist erfindungsgemäß dadurch gekennzeichnet, dass wenigstens zwei Teilstücke der Zug-/Druckstange über wendelförmig ausgebildete Endbereiche miteinander verbindbar sind.

Durch die erfindungsgemäße Ausbildung der Zug-/Druckstange ist es auf konstruktiv einfache Weise möglich, die Axialbewegung der Zug-/Druckstange in eine Rotationsbewegung der Zug-/Druckstange umzuwandeln, was insbesondere für medizinische Instrumente vorteilhaft ist, deren Maulteile seitlich abgewinkelt zur Längsachse des Schaftes ausgerichtet sind.

Um die in die Zug-/Druckstange übertragene Drehbewegung direkt und möglichst ohne eine Verschlechterung der Kraftübertragung auf das Werkzeug übertragen zu können, wird vorgeschlagen, dass das verstellbare Maulteil am distalen Ende der Zug-/Druckstange gelagert ist.

Gemäß der Erfindung ist die wendelförmige Führungsbahn an der Zug-/Druckstange ausgebildet und die Zug-/Druckstange mehrteilig ausgebildet, wobei wenigstens zwei Teilstücke der Zug-/Druckstange über wendelförmig ausgebildete Endbereiche miteinander verbindbar sind. Diese erfindungsgemäße Ausgestaltungsform, bei der zwei Teilstücke der Zug-/Druckstange über wendelförmig ausgebildete Endbereiche miteinander verschraubt sind, stellt eine besonders platzsparende und konstruktiv einfache Möglichkeit der erfindungsgemäßen Bewegungsumsetzung dar.

Mit einer praktischen Ausführungsform der Erfindung wird vorgeschlagen, dass die Zug-/Druckstange zweiteilig ausgebildet ist, wobei das proximale Teilstück am verschwenkbaren Griffteil der Handhabe festlegbar ist und am distalen Ende des distalen Teilstücks das verstellbare Maulteil des Werkzeugs angeordnet ist.

Um zu verhindern, dass bei der mehrteiligen Ausgestaltung der Zug-/Druckstange das mit dem verstellbaren Maulteil versehene Teilstück der Zug-/Druckstange zusätzlich zu der eingeleiteten Drehbewegung auch in Axialrichtung verlagert werden kann, ist der hohle Schaft vorzugsweise distalseitig verschließbar.

Weiterhin wird mit der Erfindung vorgeschlagen, dass das mit der Handhabe verbundene Teilstück der Zug-/Druckstange unverdrehbar um seine Längsachse am verschwenkbaren Griffteil der Handhabe gelagert ist, um die Drehbewegung der Zug-/Druckstange ausschließlich auf das mit dem verstellbaren Maulteil versehenen Teilstück der Zug-/Druckstange zu übertragen.

Weitere Merkmale und Vorteile der Erfindung ergeben sich anhand der Beschreibung der zugehörigen Zeichnung, in der zwei Ausführungsbeispiele eines erfindungsgemäßen medizinischen Instruments nur beispielhaft schematisch dargestellt sind. In der Zeichnung zeigt:
- Fig. 1: eine Seitenansicht eines erfindungsgemäßen medizinischen Instruments;
- Fig. 2: eine ausschnittweise perspektivische Explosionsdarstellung des distalen Endes eines medizinischen Instruments gemäß einer ersten erfindungsgemäßen Ausführungsform;
- Fig. 3: eine Draufsicht auf die Ausführungsform gemäß Fig. 2, jedoch im zusammengesetzten Zustand;
- Fig. 4: eine Darstellung gemäß Fig. 2, jedoch eine zweite erfindungsgemäße Ausführungsform darstellend;
- Fig. 5: eine perspektivische Ansicht der Ausführungsform gemäß Fig. 4, jedoch im zusammengesetzten Zustand und
- Fig.6: eine perspektivische Ansicht gemäß Fig. 5, jedoch eine nicht erfindungsgemäße Ausführungsform darstellend.

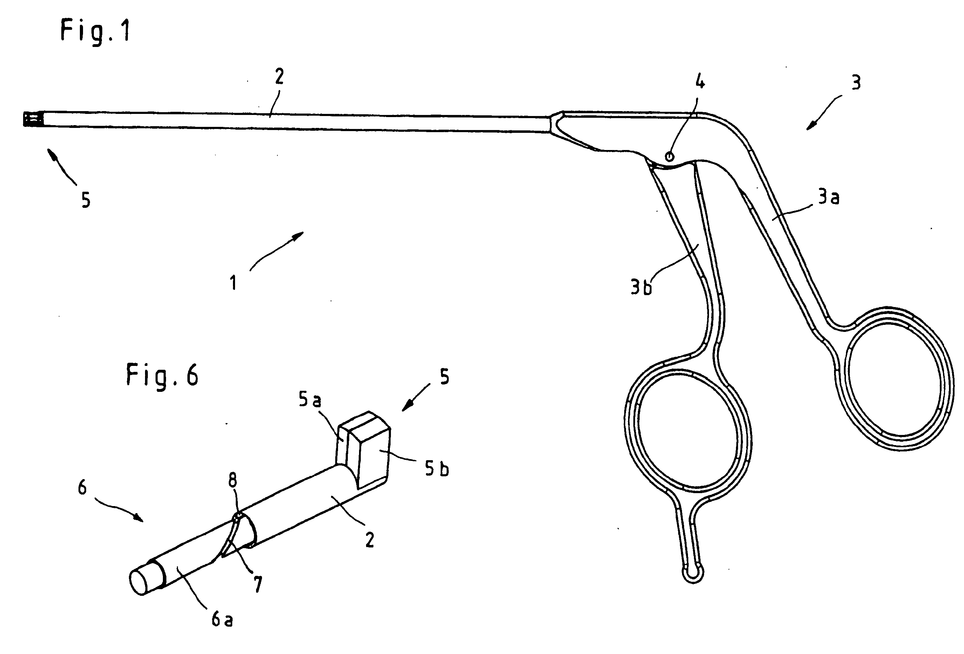

Die Abbildung Fig. 1 zeigt eine Seitenansicht eines medizinischen Instruments 1, dessen Kraftübertragungsmechanismus vielseitig verwendet werden kann, wie beispielsweise für Stanzen, Scheren, Nadelhalter, Fassinstrumente und dergleichen.

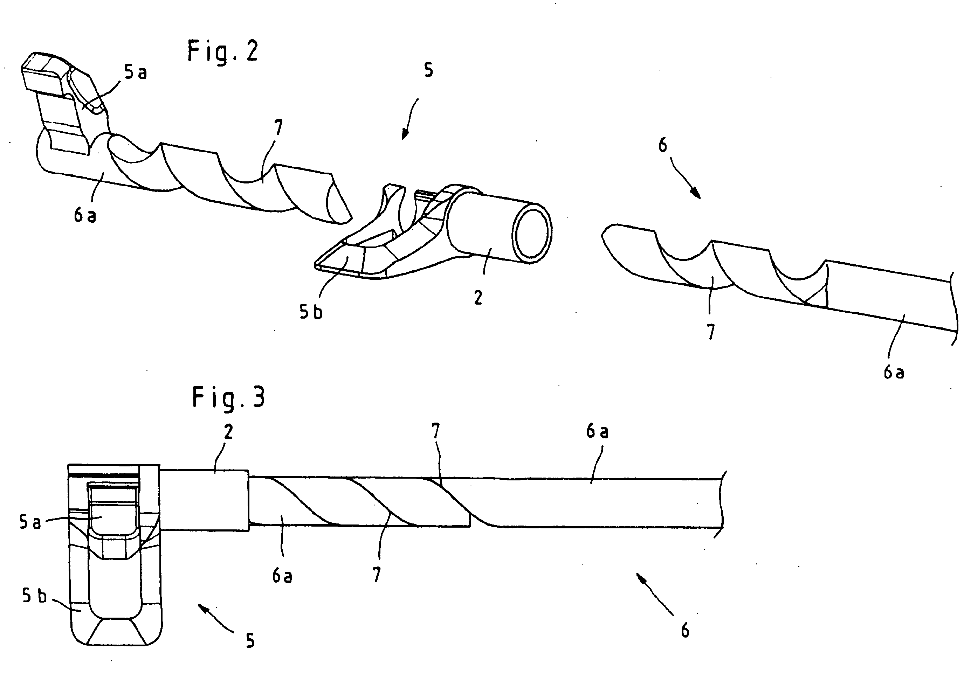

Das medizinische Instrument 1 besteht im wesentlichen aus einem hohlen Schaft 2, an dessen proximalem Ende eine aus einem starren Griffteil 3a und einem gegenüber dem starren Griffteil 3a um eine Schwenkachse 4 verschwenkbaren Griffteil 3b bestehende Handhabe 3 angeordnet ist. Am distalen Ende des Schaftes 2 ist ein Werkzeug 5 angeordnet, welches beim dargestellten Ausführungsbeispiel (Fig. 2 und 3) aus einem verschwenkbaren Maulteil 5a und einem starr mit dem Schaft 2 verbundenen Maulteil 5b besteht.

Die Darstellungen der Abbildungen Fig. 2 bis 6 zeigen, dass die Maulteile 5a, 5b des Werkzeugs 5 bezüglich der Längsachse des Schaftes 2 seitlich abwinkelt ausgebildet sind. Bei den dargestellten Ausführungsbeispielen sind die Maulteile 5a, 5b jeweils rechtwinklig zur Längsachse des Instrumentenschaftes ausgerichtet. Andere Winkellagen zwischen 0° und 90° und auch darüber hinaus sind jedoch auch praktikabel.

Durch die Abwinklung des Werkzeugs 5 gegenüber dem Schaft 2 ist es möglich, mit einem solchermaßen ausgebildeten medizinisches Instrument 1 auch schwer und nicht auf einem direkten geradlinigen Weg zugängliche Operationsgebiete zu erreichen.

Wie weiterhin aus den Darstellungen gemäß Fig. 2 bis 6 ersichtlich, ist das verschwenkbare Maulteil 5a am distalen Ende einer Zug-/Druckstange 6 gelagert, welche in dem hohlen Schaft 2 gelagert proximalseitig so mit dem verschwenkbaren Griffteil 3b der Handhabe 3 verbunden ist, dass durch das Verschwenken des Griffteils 3b das verschwenkbare Maulteil 5a von einer geschlossenen Stellung in eine geöffnete Stellung bzw. umgekehrt überführt werden kann.

Um die über die Betätigung des verschwenkbaren Griffteils 3b der Handhabe 3 auf die Zug-/Druckstange 6 übertragene Verschiebung in Axialrichtung in eine Drehbewegung zum Verschwenken des Maulteils 5a des Werkzeugs 5 umwandeln zu können, ist an der Zug-/Druckstange 6 eine wendelförmige Führungsbahn 7 ausgebildet.

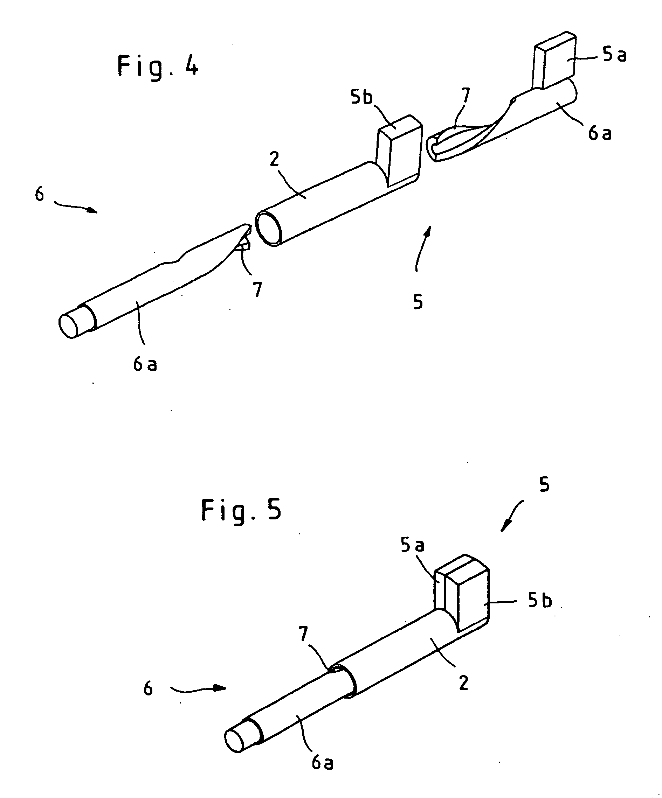

Bei den in den Abbildungen Fig. 2 bis 5 dargestellten Ausführungsformen sind die Zug-/Druckstangen 6 jeweils mehrteilig, aus einzelnen Teilstücken 6a bestehend aufgebaut. Die einander zugewandten Enden der Teilstücke 6a sind als wendelförmige so ausgebildet, dass jeweils zwei wendelförmige Führungsbahnen 7 ineinander verschraubbar sind, wie dies den Abbildungen Fig. 3 und 5 zu entnehmen ist. Das proximale Teilstück 6a der Zug-/Druckstange 6 kann ergänzend und/oder alternativ zu der dargestellten Ausführungsform in Abhängigkeit von der jeweils zu verwendenden Handhabe 3 proximalseitig beliebig verlängert und ausgeformt werden

Im zusammengesetzten Zustand wird das distale Ende des hohlen Schaftes 2 verschlossen, um eine Axialverschiebung des mit dem verschwenkbaren Maulteil 5a versehenen Teilstück 6a der Zug-/Druckstange 6 zu verhindern. Sobald über das verschwenkbare Griffteil 3b der Handhabe 3 die Zug-/Druckstange 6 in Axialrichtung im hohlen Schaft 2 verschoben wird, bewirkt diese Axialverlagerung des proximalen Teilstücks 6a der Zug-/Druckstange 6 ein ineinander- oder auseinaderschrauben der wendelförmigen Führungsbahnen 7 der beiden Teilstücke 6a der Zug-/Druckstange 6. Da das proximale Teilstück 6a der Zug-/Druckstange 6 verdrehfest am verschwenkbaren Griffteil 3b der Handhabe 3 gelagert ist, bewirkt das Ineinander- oder Auseinaderschrauben der wendelförmigen Führungsbahnen 7 der beiden Teilstücke 6a, dass das distale, mit dem verschwenkbaren Maulteil 5a versehene Teilstück 6a der Zug-/Druckstange 6 eine Rotationsbewegung um seine Längsachse ausführt, wodurch das verschwenkbaren Maulteil 5a zwischen einer geöffneten und einer geschlossenen Stellung verstellbar ist.

Die in den Abbildungen Fig. 2 und 4 dargestellten Ausführungsbeispiele unterscheiden sich dadurch voneinander, dass die beiden wendelförmigen Führungsbahnen 7 der Teilstücke 6a der Zug-/Druckstange 6 bei der Ausführungsform gemäß Fig. 2 und 3 eineinhalb Gewindegänge aufweist, während bei der Ausführungsform gemäß Fig. 4 und 5 die Führungsbahnen 7 jedes Teilstücks 6a nur einen halben Gewindegang aufweisen.

Alternativ zu den in den Abbildungen Fig. 2 bis 5 dargestellten Ausführungsformen zur Ausbildung bzw. Anordnung der wendelförmigen Führungsbahnen 7 ist es auch möglich, jedoch nicht erfindungsgemäß, die wendelförmigen Führungsbahnen 7 als wendelförmige Nut in der Mantelfläche der Zug-/Druckstange 6, wie dies in Fig. 6 dargestellt ist, oder als wendelförmige Nut an der Innenseite des hohlen Schaftes 2 auszubilden, wobei zur Erzeugung der Rotationsbewegung an der Innenseite des hohlen Schaftes 2 oder auf der Mantelfläche der Zug-/Druckstange 6 starre Widerlager 8 angeordnet sind, die mit den jeweiligen wendelförmige Nuten zusammenwirken.

Bei der in Fig. 6 dargestellten Form ist das Widerlager 8 als an der Innenseite des hohlen Schaftes 2 angeordneter und in die wendelförmige Nut eingreifender Bolzen ausgebildet.

Voraussetzung bei dieser Art der Ausgestaltung ist, dass die Zug-/Druckstange 6 frei drehbar um ihre Längsachse am verschwenkbaren Griffteil 3b der Handhabe 3 gelagert ist, damit sich die Zug-/Druckstange 6 beim Anlaufen gegen das jeweilige Widerlager aufgrund der Betätigung des verschwenkbaren Griffteil 3b um ihre Längsachse drehen kann.

Ein wie voran beschriebenes medizinisches Instrument zeichnet sich dadurch aus, dass es bei einfachem konstruktivem Aufbau und einfacher Handhabung eine gleichmäßige und sichere Umsetzung der durch das verschwenkbare Griffteil 3b auf die Zug-/Druckstange 6 übertragenen Axialbewegung in eine Rotationsbewegung des verschwenkbaren Maulteils 5a bei weitestgehend spielfreier Kraftübertragung gewährleistet.

### Bezugszeichenliste

- 1: medizinisches Instrument
- 2: Schaft
- 3: Handhabe
- 3a: starres Griffteil
- 3b: verschwenkbares Griffteil
- 4: Schwenkachse
- 5: Werkzeug
- 5a: verschwenkbares Maulteil
- 5b: starres Maulteil
- 6: Zug-/Druckstange
- 6a: Teilstück
- 7: Führungsbahn
- 8: Widerlager

## Patentansprüche

1. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (5a, 5b) bestehendes Werkzeug (5) angeordnet ist, wobei mindestens ein Maulteil (5a) des Werkzeugs (5) zum Öffnen und Schließen über ein verschwenkbar ausgebildetes Griffteil (3b) der Handhabe (3) gegenüber dem mindestens einen anderen Maulteil (5b) des Werkzeugs (5) verstellbar ist und das mindestens eine verstellbare Maulteil (5a) und das entsprechende, zum Verstellen des Maulteils (5a) dienende Griffteil (3b) der Handhabe (3) über eine axial verschiebbare in dem hohlen Schaft (2) gelagerte Zug-/Druckstange (6) miteinander verbunden sind, wobei die in dem hohlen Schaft (2) gelagerte Zug-/Druckstange (6) über mindestens eine wendelförmige Führungsbahn (7) in eine Rotationsbewegung um ihre Längsachse überführbar ist, wobei
die Zug-/Druckstange (6) mehrteilig ausgebildet ist, wobei ein Teilstück (6a) unverdrehbar um seine Längsachse am verschwenkbaren Griffteil (3b) gelagert ist und ein anderes Teilstück (6a) um seine Längsachse verdrehbar mit dem verstellbaren Maulteil (5a) gekoppelt ist **dadurch gekennzeichnet, dass** wenigstens zwei Teilstücke (6a) der Zug-/Druckstange (6) über wendelförmig ausgebildete Endbereiche miteinander verbindbar sind.

2. Medizinisches Instrument nach Anspruch 1, **dadurch gekennzeichnet, dass** das verstellbare Maulteil (5a) am distalen Ende der Zug-/Druckstange (6) gelagert ist.

3. Medizinisches Instrument nach Anspruch 1 oder 2, **dadurch gekennzeichnet, dass** die Zug-/Druckstange (6) zweiteilig ausgebildet ist, wobei das proximale Teilstück (6a) am verschwenkbaren Griffteil (3b) der Handhabe (3) festlegbar ist und am distalen Ende des distalen Teilstücks (6a) das verstellbare Maulteil (5a) des Werkzeugs (5) angeordnet ist.

4. Medizinisches Instrument nach einem der Ansprüche 1 bis 3, **dadurch gekennzeichnet, dass** der hohle Schaft (2) distalseitig verschließbar ist.

5. Medizinisches Instrument nach einem der Ansprüche 1 bis 4, **dadurch gekennzeichnet, dass** die Maulteile (5a, 5b) seitlich abgewinkelt zur Längsachse des Schaftes (2) ausgerichtet sind.

## Claims

1. Medical instrument with a hollow shaft (2), at whose proximal end there is a handle (3) consisting of at least two grip parts (3a, 3b) and at whose distal end there is a tool (5) consisting of at least two jaw parts (5a, 5b), where at least one jaw part (5a) of the tool (5) is adjustable relative to the at least one other jaw part (5b) of the tool (5) for opening and closing via a pivotable grip part (3b) of the handle (3), and where the at least one adjustable jaw part (5a) and the corresponding grip part (3b) of the handle (3), serving to adjust the jaw part (5a), are connected to each other via an axially displaceable pull/push rod (6) mounted in the hollow shaft (2), where the pull/push rod (6) mounted in the hollow shaft (2) can be set into a rotation movement about its longitudinal axis via at least one helical guide track (7), where the pull/push rod (6) has a multi-part design, one constituent part (6a) being mounted non-rotatably about its longitudinal axis on the pivotable grip part (3b), and another constituent part (6a) being coupled to the adjustable jaw part (5a) so as to be able to rotate about its longitudinal axis, **characterized in that** at least two constituent parts (6a) of the pull/push rod (6) can be connected to each other via helically configured end areas.

2. Medical instrument according to Claim 1, **characterized in that** the adjustable jaw part (5a) is mounted on the distal end of the pull/push rod (6).

3. Medical instrument according to Claim 1 or 2, **characterized in that** the pull/push rod (6) is designed in two parts, the proximal constituent part (6a) being able to be secured on the pivotable grip part (3b) of the handle (3), and the adjustable jaw part (5a) of the tool (5) being arranged on the distal end of the distal constituent part (6a).

4. Medical instrument according to one of Claims 1 to 3, **characterized in that** the hollow shaft (2) can be closed at the distal end.

5. Medical instrument according to one of Claims 1 to 4, **characterized in that** the jaw parts (5a, 5b) are oriented laterally at an angle to the longitudinal axis of the shaft (2).

## Revendications

1. Instrument médical, comprenant un corps allongé creux (2) à l'extrémité proximale duquel est agencée une poignée de manoeuvre (3) constituée d'au moins deux pièces de préhension (3a, 3b), et à l'extrémité distale duquel est agencé un outil (5) constitué d'au moins deux pièces de mâchoire (5a, 5b), au moins une pièce de mâchoire (5a) de l'outil (5) pouvant être déplacée par rapport à ladite au moins une autre pièce de mâchoire (5b) de l'outil (5), pour l'ouverture et la fermeture, par l'intermédiaire d'une pièce de préhension pivotante (3b) de la poignée de manoeuvre (3), et ladite au moins une pièce de mâchoire déplaçable (5a) et la pièce de préhension (3b) correspondante de la poignée de manoeuvre (3), qui sert à déplacer la pièce de mâchoire (5a), étant reliées mutuellement par l'intermédiaire d'une tige de traction/compression (6) montée axialement coulissante dans le corps allongé creux (2), la tige de traction/compression (6) montée dans le corps allongé creux (2) pouvant être mise en un mouvement de rotation autour de son axe longitudinal par l'intermédiaire d'au moins une voie de guidage (7) en forme hélice, et la tige de traction/compression (6) étant d'une configuration en plusieurs parties, une partie (6a) étant montée non rotative autour de son axe longitudinal sur la pièce de préhension pivotante (3b), et une autre partie (6a) étant couplée, de manière rotative autour de son axe longitudinal, à la pièce de mâchoire déplaçable (5a),
**caractérisé en ce qu'**au moins deux parties (6a) de la tige de traction/compression (6) peuvent être reliées l'une à l'autre par l'intermédiaire de zones d'extrémité d'une configuration en forme d'hélice.

2. Instrument médical selon la revendication 1, **caractérisé en ce que** la pièce de mâchoire déplaçable (5a) est montée à l'extrémité distale de la tige de traction/compression (6).

3. Instrument médical selon la revendication 1 ou 2, **caractérisé en ce que** la tige de traction/compression (6) est réalisée en deux parties, la partie proximale (6a) pouvant être fixée à la pièce de préhension pivotante (3b) de la poignée de manoeuvre (3), et à l'extrémité distale de la partie distale (6a) est agencée la pièce de mâchoire déplaçable (5a) de l'outil (5) .

4. Instrument médical selon l'une des revendications 1 à 3, **caractérisé en ce que** le corps allongé creux (2) peut être fermé du côté distal.

5. Instrument médical selon l'une des revendications 1 à 4, **caractérisé en ce que** les pièces de mâchoire (5a, 5b) sont orientées de façon coudée latéralement par rapport à l'axe longitudinal du corps allongé (2).
